# EUROPÄISCHE PATENTSCHRIFT

(11) **EP 0 659 277 B1**
(45) Veröffentlichungstag und Bekanntmachung des Hinweises auf die Patenterteilung: **20.11.1996**
(21) Anmeldenummer: 93919227.4
(22) Anmeldetag: 31.08.1993
(51) Int. Cl.: G01N 33/74, G01N 33/543, G01N 33/03, G01N 33/12

(54) **VERFAHREN ZUR BESTIMMUNG VON ANDROSTENON-GEHALTEN IN FETTGEWEBEN**
PROCESS FOR DETERMINING THE ANDROSTENONE CONTENT OF ADIPOSE TISSUES
PROCEDE DE DETERMINATION DE LA TENEUR EN ANDROSTENONE DE TISSUS ADIPEUX

(30) Priorität: 08.09.1992 DE 4229904
(43) Veröffentlichungstag der Anmeldung: 28.06.1995
(73) Patentinhaber: RIEDEL-DE HAEN AKTIENGESELLSCHAFT, D-30926 Seelze (DE)
(72) Erfinder: CLAUS, Rolf, Paul, D-70599 Stuttgart (DE); DEHNHARD, Martin, D-71111 Waldenbuch (DE)
(74) Vertreter: Geissler, Bernhard, Dr. jur., Dipl.-Phys.
(86) Internationale Anmeldenummer: EP9302350
(87) Internationale Veröffentlichungsnummer: WO9406018

(56) Entgegenhaltungen:
- ARCHIV FÜR LEBENSMITTELHYGIENE Bd. 39, Nr. 4 , Juli 1988 , HANNOVER, DE Seiten 87 - 90 R. CLAUS, G. MAHLER UND E. MÜNSTER 'Determination of the boar steroid 5a-androst-16-en-3-one in adipose tissue of pigs with a rapid microtitre plate enzyme-immunoassay (MTE)' in der Anmeldung erwähnt
- CHEMICAL ABSTRACTS, vol. 85, no. 21, 22. November 1976, Columbus, Ohio, US; abstract no. 158023, KAUFMANN, G.; RITTER, F.; SCHUBERT, K. 'Quantitative determination of the boar taint substance 5.alpha-androst-16-en-3-one in fat' Seite 386 ;
- CHEMICAL ABSTRACTS, vol. 111, no. 7, 14. August 1989, Columbus, Ohio, US; abstract no. 55921, EDELHÄUSER, MANFRED 'A method for the rapid determination of the boar taint steroid androstenone [in the adipose tissue of swine]' Seite 608 ;
- CHEMICAL ABSTRACTS, vol. 83, no. 1, 7. Juli 1975, Columbus, Ohio, US; abstract no. 4049, KAUFMANN,G.; SCHUBERT,K. 'Method for determination of the odorous substance 5.alpha-androst-16-en-3-one in boar fat' Seite 368 ;
- JOURNAL OF AGRICULTURAL AND FOOD CHEMISTRY Bd. 38, Nr. 1 , Januar 1990 , WASHINGTON US Seiten 331 - 335 MOHAMED M. ABOUZIED ET AL. 'Monoclonal Antibody-Based Enzyme-Linked Immunosorbent Assay for C19-delta16-Steroids in Sera of Boar Pigs'
- ARCHIV FÜR LEBENSMITTELHYGIENE, Band 39, Nr. 4, Juli 1988, Hannover (DE); R. CLAUS et al., Seiten 87-90/
- CHEMICAL ABSTRACTS, Band 85, Nr. 21, 22 November 1976, Columbus, OH (US); G. KAUFMANN et al., Seite 386, Nr. 158023/
- CHEMICAL ABSTRACTS, Band 111, Nr. 7, 14 August 1989, Columbus, OH (US); M. EDELHÄUSER, Seite 608, Nr. 55921/
- CHEMICAL ABSTRACTS, Band 83, Nr. 1, 7. Juli 1975, Columbus, Ohio, US; G. KAUFMANN et al., Seite 368, Nr. 4049/
- JOURNAL OF AGRICULTURAL & FOOD CHEMISTRY, Band 38, Nr. 1, Januar 1990, Washington, DC (US); M.M. ABOUZIED et al., Seiten 331-335/

## Beschreibung

Die Erfindung betrifft ein Verfahren zur Bestimmung von Androstenon-Gehalten in Fettgeweben sowie eine Vorrichtung zur Durchführung dieses Verfahrens.

5α-Androst-16-en-3-on, das für den Geschlechtsgeruch des Ebers verantwortlich ist und einen intensiv urinartigen Geruch aufweist, wird im Fettgewebe der Tiere angereichert. Diese Anreicherung im Fett ist unabhängig von der anatomischen Lage des Fettgewebes und betrifft gleichermaßen z.B. subkutanes Fett (Speck), intermuskuläres Fett, intramuskuläres Fett und Organfett. Damit entsteht das erhebliche Problem, daß Fleisch von Ebern, insbesondere beim Erwärmen im Rahmen der Zubereitung, einen sensorisch äußerst störenden Uringeruch verursachen kann. Andererseits zeigen Eber aufgrund der anabolen Wirkung der neben Androstenon ebenfalls im Hoden gebildeten Hormone erhebliche Wachstumsvorteile. Die Zulassung der Ebermast innerhalb der EG verlangt nach einer Untersuchungsmöglichkeit, um routinemäßig solche Eber zu entdecken und aussondern zu können, bei denen, aufgrund fortgeschrittener Pubertätsentwicklung, die Geschlechtsgerucheinlagerung im Fett bereits ein sehr hohes Niveau erreicht hat. Als noch tolerierbarer Grenzwert für den Androstenon-Gehalt werden derzeit 0,5 µg/g Fettgewebe diskutiert.

Zwar sind bisher schon zuverlässige Meßverfahren, insbesondere auf enzymimmunologischer Basis, zur Bestimmung von Androstenon im Fettgewebe bekannt, jedoch lassen die bekannten Verfahren allenfalls stichprobenartige Labor-Untersuchungen, keinesfalls jedoch eine kontinuierliche kontrolle parallel zum Schlachtband aufgrund des sehr hohen Zeitbedarfs der Analysen zu.

Aufgabe der Erfindung ist es, die bekannten Meßverfahren so weit zu verbessern, daß diese in einer wesentlich kürzeren Zeit durchzuführen sind und möglichst auch in einem technisch einfachen, automatisierbaren Verfahren angewendet werden können.

Diese Aufgabe wird bei einem Verfahren der eingangs beschriebenen Art erfindungsgemäß dadurch gelöst, daß das Bestimmungsverfahren folgende Schritte umfaßt:
a) Verflüssigung einer Fettgewebeprobe durch Erwärmen auf eine vorgegebene Temperatur im Bereich von 45 bis 60°C;
b) Mischen einer definierten Menge des flüssigen Fetts mit einem wasserlöslichen Lösemittel für das Androstenon bei der Temperatur des flüssigen Fetts;
c) Abkühlen der Fett/Lösemittelmischung auf eine vorgegebene Temperatur, bei der wesentliche Anteile des im Lösemittel gelösten Fetts einerseits aus der Lösung ausgeschieden werden und andererseits jedoch der überwiegende Teil des in der Lösemittelphase gelösten Androstenon im Lösemittel gelöst verbleibt;
d) Entnahme einer definierten Menge der androstenonhaltigen Lösemittelphase und Verdünnung in einem vorgegebenen Verhältnis mit einer für das verwendete Nachweisverfahren verträglichen wäßrigen Pufferlösung;
e) Ermittlung des Androstenon-Gehalts der Lösemittel/Putterlösungsphase mittels an sich bekannter kompetitiver immunologischer Nachweisreaktionen.

Ein besonders geeignetes nachweisverfahren stellt das Meßverfahren, das aus der Publikation von R. Claus, G. Mahler und E. Münster in "ARCHIV FÜR LEBENSMITTELHYGIENE", Jahrgang 39, Heft 4/1988, Seiten 87-90, bekannt wurde, dar.

Das genannte immunologische Meßverfahren beruht darauf, daß in einem Testsystem eine definierte Menge spezifischer, gegen die zu messende Substanz (hier das Androstenon) erzeugter Antikörper vorgelegt wird. Zur besseren Handhabung der Antikörper werden diese auf einer Aufnahmeplatte, insbesondere einer Mikrotiterplatte, immobilisiert. Die so vorbereitete Aufnahmeplatte wird mit einem enzymmarkierten Androstenon und der das freie, zu bestimmende Androstenon enthaltenden, lösemittelhaltigen Pufferlösung inkubiert. Nach Ablauf der vorgegebenen Inkubationszeit wird dekantiert und die Aufnahmeplatte von nicht an die Antikörper gebundenem Androstenon befreit.

Der Anteil an enzymmarkiertem Androstenon, der an den immobilisierten Antikörpern gebunden an der Aufnahmeplatte vorliegt, hängt direkt von dem Anteil an freiem Androstenon ab, der in der lösemittelhaltigen Pufferlösung vorhanden war. Die nach dem eben beschriebenen Verfahrensschritt meßbare Enzymaktivität resultiert von den an den Antikörpern gebundenen enzymmarkierten Androstenon-Anteilen und ist damit ein Maß, in welchem Umfang das enzymmarkierte Androstenon durch freies, durch die lösemittelhaltige Pufferlösung eingeführtes Androstenon verdrängt worden ist.

Anhand von bekannten enzymatischen Reaktionen, beispielsweise anhand von enzymatischen Oxidations- oder Reduktionsreaktionen mit Farbstoffen, läßt sich dann eine leicht meßbare Größe für das Vorhandensein von enzymmarkiertem Androstenon auf der Aufnahmeplatte erhalten.

Im Detail ist diese Methode noch anhand eines Beispiels weiter unten erläutert.

Selbstverständlich kommen auch alternative kompetitive immunologische Nachweisreaktionen für das erfindungsgemäße Verfahren in Frage.

Für die Anwendbarkeit des erfindungsgemäßen Verfahrens in automatisierten Meßanlagen, die parallel zum Schlachtband betrieben werden können, ist maßgebend, daß ein Minimum an Probenaufbereitungsschritten verwendet wird und daß insbesondere langwierige Trocknungs-, Einengungs- oder sonstige Destillationsschritte vermieden sind.

Das Vermeiden solcher Schritte fördert auch die Verläßlichkeit des Verfahrens, da die Androstenone neben ihrer extrem guten Fettlöslichkeit auch eine relativ hohe Flüchtigkeit besitzen, weshalb zum einen möglichst bei niedrigen Temperaturen gearbeitet wird und zum anderen eine Methode, die Trocknungs- und Destillationsschritte vermeidet, die Verläßlichkeit der Analysenergebnisse steigert.

Vorzugsweise wird das Lösemittel so ausgewählt, daß es neben guten Löseeigenschaften für das nachzuweisende Androstenon im Temperaturbereich von ca. 20 bis 60°C einen Temperaturkoeffizienten für die Fettlöslichkeit aufweist, welcher größer ist als der Temperaturkoeffizient für die Androstenon-Löslichkeit in diesem Temperaturbereich. Dies erlaubt durch einen Abkühlungsschritt nach der Mischung der flüssigen Fettprobe mit dem Lösemittel in dem Lösemittel gelöste Fettanteile effektiv abzuscheiden, so daß später bei dem Schritt der Mischung der Lösemittelphase mit der wäßrigen Pufferlösung im wesentlichen keine Abscheidung von Fetttröpfchen oder allgemein einer Fettphase mehr beobachtet wird. Gleichzeitig bleibt aber bei der Abkühlung der Mischung das im Lösemittel vorhandene gelöste Androstenon in der Lösung erhalten und steht somit für die spätere Nachweisreaktion zur Verfügung.

Damit möglichst wenig Störungen mit der späteren im Nachweisreaktionsystem verwendeten Enzymreaktion auftreten, sollte das Lösungsmittel vorzugsweise allenfalls einen geringen Einfluß auf die Enzymaktivität haben.

Ferner ist erwünscht, daß das Lösemittel kaum Einfluß auf die Antigen/Antikörperreaktionen des Nachweisreaktionssystems aufweist, um die Selektivität und Empfindlichkeit des Nachweissystems nicht zu vermindern.

Als besonders geeignete Lösemittel haben sich methanolhaltige Lösemittel erwiesen, und es hat sich überraschenderweise herausgestellt, daß insbesondere reines Methanol vorzüglich für das erfindungsgemäße Verfahren geeignet ist. Überraschenderweise nimmt das in den Folgeschritten weiterverschleppte Methanol kaum Einfluß auf die Enzymaktivität im Nachweisreaktionssystem und läßt auch das Antigen/Antikörperreaktionssystem im wesentlichen unbeeinflußt.

Dies trifft insbesondere dann zu, wenn das Mischungsverhältnis der flüssigen Fettprobe zum Lösemittel im Bereich von 1:10 bis 0,1:10 (Volumenverhältnisse) gewählt wird. Der angegebene Bereich des Mischungsverhältnisses von flüssiger Fettprobe zu Lösemittel stellt sicher, daß zum einen ausreichend Fettphase zur Extraktion des Androstenons zur Verfügung steht, so daß im Folgeschritt mit ausreichend geringen Lösungsmittelphasenanteilen gearbeitet werden kann. Selbstverständlich hängen die Grenzen, innerhalb derer die Erfindung praktikabel ist, nicht unwesentlich von der Wahl des Lösungsmittels ab und selbstverständlich darüberhinaus auch von der Wahl des Nachweisreaktionssystems.

Als besonders gut geeigneter Arbeitsbereich hat sich ein Mischungsverhältnis von flüssiger Fettprobe zu Lösungsmittel im Bereich von 0,2:10 bis 0,5:10 erwiesen.

Ein weiterer wichtiger Schritt im Hinblick auf die mögliche Störung bzw. Beeinflussung von enzymatischen Reaktionen bzw. von immunologischen Reaktionen ist das Verhältnis der Verdünnung der Lösungsmittelphase mit der Pufferlösung. Dies wird bevorzugt im Volumenverhälnis von 20:80 bis 5:95 vorgenommen, da so zum einen sichergestellt ist, daß nicht zuviel Lösungsmittel in das Reaktionsmedium eingeschleppt wird, in dem dann die Antigen/Antikörperreaktion stattfindet, und zum anderen ist sichergestellt, daß ausreichend Androstenon für die Konkurrenzsituation mit dem enzymmarkierten Androstenon zur Verfügung steht, so daß letztendlich die geforderte Nachweisgrenze eingehalten werden kann.

Insbesondere im Hinblick auf den Verdünnungsschritt wird bereits beim Abkühlungsschritt die Endtemperatur der Probe nach der Abkühlung so gewählt, daß das zunächst während dem Vermischen mit der flüssigen Fettprobe in dem Lösemittel gelöste Fett in einem solchen Umfang aus dem Lösemittel abgeschieden wird, daß im nachfolgenden Verdünnungsschritt (Schritt d)) eine im wesentlichen fettphasenfreie Lösung erhalten wird. Falls bei dieser Mischung von Lösemittel und wäßriger Pufferlösung eine Ausscheidung einer Fettphase in größerem Ausmaß vorkommen sollte, ist zu gewärtigen, daß dieser Vorgang die späteren Nachweisreaktionen, insbesondere auch die photometrische Bestimmung der Enzymaktivität, hindert.

Das bereits zuvor in Einzelheiten beschriebene Nachweisverfahren für Androstenone, das in der Veröffentlichung in "ARCHIV FÜR LEBENSMITTELHYGIENE" 1988, Seiten 87-90, veröffentlicht ist, wurde insbesondere in einem Punkt modifiziert, nämlich in der Temperatur, bei der die immobilisierten Antikörper mit dem enzymmarkierten und freien Androstenon inkubiert werden.

Das im Schritt e) des erfindungsgemäßen Verfahrens bevorzugte Nachweisverfahren läßt sich durch folgende Schritte charakterisieren:
(1) daß eine Aufnahmeplatte mit einem Antikörperserum beschichtet wird und die Antikörper an der Aufnahmeplatte immobilisiert werden, wobei die Antikörper spezifisch mit Androstenonen, insbesondere 5α-Androst-16-en-3-on, reagieren;
(2) daß die immobilisierten Antikörper mit einer definierten Menge eines enzymmarkierten Androstenons und mit einem vorgegebenen Volumen der in Schritt d) erhaltenen lösemittelhaltigen Pufferlösung bei einer Temperatur von ca. 42 bis 48°C, vorzugsweise bei ca. 45°C, für eine vorgegebene Zeitspanne gleichzeitig inkubiert werden;
(3) daß das nichtgebundene enzymmarkierte und freie Androstenon entfernt und die beschichtete Aufnahmeplatte gewaschen wird;
(4) daß eine ein Substrat enthaltende Pufferlösung auf die Aufnahmeplatte gegeben wird, wobei das Substrat mit dem Markerenzym eine Enzymreaktion eingeht;
(5) daß die Enzymreaktion für eine vorbestimmte Zeitspanne bei einer vorgegebenen Temperatur durchgeführt wird und danach mit einem geeigneten Reaktionsmittel abgestoppt wird; und
(6) daß der Reaktionsumsatz der enzymatischen Reaktion, vorzugsweise photospektrometrisch, bestimmt wird.

Hier hat sich herausgestellt, daß entgegen der früheren Praxis, bei der diese Inkubation bei 37°C vorgenommen wurde, trotz der hohen Flüchtigkeit des Androstenons eine bedeutend höhere Temperatur Anwendung finden kann, nämlich in einem Temperaturbereich von ca. 42 bis 48°C. Vorzugsweise wird eine Temperatur von ca. 45°C eingehalten, bei der unter den vordefinierten Voraussetzungen bei ausreichender Nachweissicherheit eine minimale Zeitspanne für das Inkubieren benötigt wird.

Ein weiterer Vorteil des erfindungsgemäßen Verfahrens ist, daß aufgrund der fehlenden Trennschritte und aufgrund der sorgfältigen Auswahl des verwendeten Lösemittels, mit der Möglichkeit dieses bis in die Antigen/Antikörperreaktionssysteme zu verschleppen, die Weiterverarbeitungen der einzelnen Lösungen und Flüssigkeitsgemische allein durch Pipettieren möglich wird. Dadurch wird die Möglichkeit geschaffen, den Transport, das Vermischen und Trennen von Phasen etc. auf Pipettierschritte zu beschränken, die leicht automatisierbar sind. Darüber hinaus fallen bei dem erfindungsgemäßen Verfahren nur geringste Mengen an zu entsorgenden flüssigen Abfallstoffen und Abwässern an. Diese sind außerdem ohne besondere Vorkehrungen zu verwerten und beispielsweise dem Abwasserkanal zuzuführen.

Eine geeignete Vorrichtung zur automatisierten Durchführung des erfindungsgemäßen Verfahrens ist durch eine an sich bekannte programmierbare und temperierbare Pipettiervorrichtung gegeben, welche zusätzlich mit einer heizbaren Probeneingangsstation ausgerüstet ist, welche die eingangsseitige Fettgewebeprobe aufnimmt und bei der gewünschten Temperatur verflüssigt.

Die Pipettiervorrichtung ist mit mehreren Probeaufnahmevorrichtungen, insbesondere Mikrotiterplatten, ausgerüstet und führt die Verfahrensschritte b), c) und d) sowie gegebenenfalls die zusätzlichen Schritte 2, 3, 4 und 5 des Probenaufbereitungsverfahrens sowie des Nachweisverfahrens unter Einhaltung definierter Temperaturbedingungen und Zeitdauern für die einzelnen Verfahrensschritte durch.

Ausgangsseitig wird die Pipettiervorrichtung mit einer Meßvorrichtung zur direkten oder indirekten Bestimmung einer den Androstenon-Gehalt der Fettgewebeprobe reflektierenden Meßgröße ausgerüstet.

Eine Kalibrierung der Meßergebnisse erfolgt vorzugsweise unter Durchführung der für die zu untersuchende Probe vorgesehenen Verfahrensschritte bei identischen Bedingungen, jedoch unter Verwendung von Schweineschmalz mit definiert zugegebenen Androstenon-Anteilen. Damit lassen sich alle vorrichtungseigenen und verfahrenstypischen Parameter und Fehler ausgleichen, da der Probengehalt nachfolgend aufgrund der mit der Eichsubstanz erhaltenen Meßkurve ermittelt wird.

Die Erfindung betrifft weiterhin die Verwendung einer Aufnahmeplatte, auf welcher eine definierte Menge gegen Androstenon spezifische Antikörper immobilisiert ist, zur Aufnahme der in Schritt d) des erfindungsgemäßen Verfahrens erhaltenen androstenonhaltigen Probe zusammen mit einem enzymmarkierten Androstenon und zur anschließenden Durchführung der kompetitiven Nachweisreaktion gemäß Schritt e) des erfindungsgemäßen Verfahrens. Bevorzugt ist dabei die Verwendung einer Aufnahmeplatte, die als Mikrotiterplatte ausgebildet ist.

Die beim erfindungsgemäßen Verfahren verwendete kompetitive immunologische Nachweisreaktion hat den Vorteil einer extrem hohen Spezifität für die zu messende Substanz. Allerdings sind für den ungestörten Ablauf dieser Nachweisreaktionen physiologische Reaktionsbedingungen sicherzustellen, weshalb den vorgeschaltenen Aufarbeitungs- und Reinigungsschritten erhebliche Bedeutung zukommt. Diese Verfahrensschritte müssen einerseits die zu messende Substanz aus der biologischen Matrix (hier Fettgewebe) isolieren und dabei sicherstellen, daß durch die leichte Flüchtigkeit von Androstenon keine Verluste an zu messender Substanz eintreten. Andererseits muß gewährleistet sein, daß keine Kontaminationen in das Nachweisreaktionssystem verschleppt werden (z.B. Reagenzien-Reste, Lipide aus der Probe, etc.), die geeignet sind, unphysiologische Situationen zu schaffen oder aber in sonstiger Weise das Meßsystem, insbesondere auch die bevorzugt verwendete photometrische Bestimmung des Reaktionsergebnisses, zu stören. Das erfindungsgemäße Verfahren schafft nun ein völlig neues Verfahren zur Probenextraktion und -reinigung mit dem Vorteil, daß Androstenon trotz seiner lipophilen Eigenschaften aus der Fettgewebeprobe in das wäßrige Meßsystem transferiert und gleichzeitig Kontaminationen mit störenden Lipiden sowie Androstenon-Verluste aufgrund dessen Flüchtigkeit vermieden werden.

Als am besten geeignet hat sich im wesentlichen reines Methanol als Lösemittel für die Extraktion aus der Fettgewebeprobe erwiesen. Nach Abkühlen des zunächst auf die Temperatur der flüssigen Fettgewebeprobe aufgeheizten Methanols erniedrigt sich die Löslichkeit der kontaminierenden Lipide, die als Fetttröpfchen den Boden des Reagenzgefäßes, insbesondere des Reagenzglases, sedimentieren bzw. an der Glasoberfläche adsorbieren. Ein aliquoter Teil der fettpartikelfreien abgekühlten Methanolphase wird mit einem darauf abgestimmten Volumen eines wäßrigen Testpuffersystems verdünnt. Dieses Gemisch kann erfindungsgemäß direkt in das enzymimmunologische Mikrotitersystem überführt werden.

Zur Erzielung besonders verläßlicher Werte ist insbesondere auch eine exakte Abstimmung zwischen den Volumina an flüssigem Fett, warmem Methanol sowie die Relation des aliquoten Teils des abgekühlten Methanols zur Testpufferlösung von besonderer Bedeutung. Nur durch die sorgfältige Abstimmung aller Volumina ist gewährleistet, daß die noch in gewissen Mengen in das Nachweisreaktionssystem gelangenden Lipid- und Methanolanteile unter den gewählten Bedingungen den Ablauf der immunologischen als auch der enzymatischen Folgereaktion nicht stören.

Dies ist besonders wichtig bei einem anzustrebenden Grenzwert von 0,5 µg/g bis 0,7 µg/g Fettgewebe. Aufgrund des neuen, erfindungsgemäßen Verfahrens und hier insbesondere durch die neue Art der Aufbereitung der Fettgewebeprobe ist im Gegensatz zu allen anderen bekannten Aufarbeitungs- und Isolierungsverfahren von nachzuweisenden Substanzen (einschließlich Androstenon) aus Geweben gesichert, daß sämtliche Schritte mit Hilfe von Pipettiervorgängen durchführbar sind. Dadurch werden einerseits insbesondere die zeitaufwendige Evaporation von Lösemitteln oder Zentrifugationsschritte vermieden und andererseits der Einsatz von prozeßgesteuerten Pipettierautomaten bzw. kommerziell verfügbaren Mikrotiterbausteinen ermöglicht.

Die Überprüfung des erfindungsgemäßen Meßverfahrens an einem Tag (Prüfung auf Intraassay-Varianz) bzw. an mehreren Tagen (Interassay-Varianz) ergab die in Tabelle 1 angegebenen Werte. Es sei in diesem Zusammenhang insbesondere auf die Varianzen im Bereich der Nachweisgrenze von 0,5 µg/g Fettgewebe hingewiesen, die bei 15 % (Interassay-Varianz) bzw. bei 10 % (Intraassay-Varianz) liegen.

Die Meßergebnisse gemäß dem erfindungsgemäßen Verfahren wurden mit Messungen gemäß dem bekannten Standard-Testverfahren (Referenzmethode gemäß "ARCHIV FÜR LEBENSMITTELHYGIENE" 1988, Seite 87-90) verglichen, und es ergab sich eine sehr hohe Übereinstimmung der Meßwerte gemäß dem erfindungsgemäßen Schnelltest oder Schnellverfahren mit denen des Referenzverfahrens (r = 0,96; p < 0,0001). Die Mittelwerte aller 124 gemessenen Proben betrugen 0,72 µg/g Fettgewebe für das Referenzverfahren bzw. 0,71 µg/g gemäß dem Schnelltest.

**TABELLE 1**

| Präzision und Intra- bzw. Interassay-Variationskoeffizienten (VK) der Androstenonmessung im Fett | | | | |
|---|---|---|---|---|
| Fettproben mit Zusätzen von Androstenon (µg/g) | Intraassay (n = 14) | | Interassay (n = 10) | |
| | Mittelwert | VK | Mittelwert | VK |
| 0,1 | 0,09 | 12 % | 0,09 | 20 % |
| 0,2 | 0,23 | 9 % | 0,23 | 14 % |
| 0,5 | 0,54 | 10 % | 0,44 | 15 % |
| 0,75 | 0,88 | 8 % | 0,72 | 9 % |
| 1,0 | 1,14 | 11 % | 1,08 | 8 % |
| 1,5 | 1,64 | 14 % | 1,67 | 16 % |

| Biologische Proben | Intraassay (n = 16) | | Interassay (n = 12) | |
|---|---|---|---|---|
| | Mittelwert | VK | Mittelwert | VK |
| Nr. 1 | 0,22 | 8 % | 0,23 | 19 % |
| Nr. 2 | 0,42 | 9 % | 0,48 | 15 % |
| Nr. 3 | 0,88 | 9 % | 0,88 | 12 % |
| Mittlerer Intraassay-VK: 10 % | | | | |
| Mittlerer Interassay-VK: 14 % | | | | |

**TABELLE 2**

| Praktikabilität | |
|---|---|
| Verfahren | Probendurchsatz je Stunde |
| konventionell (nicht automatisierbar!) (Claus et al. 1988) | 25 |
| | |
| erfindungsgemäßes Verfahren (manuell) | 75 |
| | |
| erfindungsgemäßes Verfahren (automatisiert) | 540 |

Tabelle 2 zeigt den Zeitvorteil, der durch die erfindungsgemäße Methode gegenüber der bekannten Referenzmethode erzielt wird. Während mit der Referenzmethode maximal 25 Proben/h bearbeitet werden können, erlaubt das erfindungsgemäße neue Bestimmungs-verfahren selbst bei manueller Ausführung die Bearbeitung von ca. 75 Proben/h. Bei Verwendung eines vollautomatisierten Meßverfahrens läßt sich der Probendurchsatz dramatisch steigern, beispielsweise mit bekannten Pipettierautomaten auf mehr als 500 Proben/h.

Im folgenden wird das erfindungsgemäße Bestimmungsverfahren im einzelnen erläutert, einschließlich einer ins einzelne gehenden Erläuterung eines bevorzugten Nachweismeßsystems.

Schema 1: Probenextraktion und Reinigung zur automatisierbaren Messung von 5α-Androst-16-en-3-on ("Geschlechtsgeruch") im Fettgewebe von Schweinen (Schritte 1 - 4 beinhalten die Probenverarbeitung, Schritte 5 - 10 die immunologische Messung).
Schritt 1: ca. 2 g Fettgewebe in der Mikrowelle erwärmen: 4 min bei 180 W
Schritt 2: 25 µl verflüssigtes Fett so abpipettieren, daß keine Abkühlung entsteht und homogenisieren in 1 ml Methanol
Schritt 3: Abkühlen auf Raumtemperatur, nach einigen Minuten abpipettieren von 100 µl und verdünnen mit 900 µl Puffer (0,04 M Natriumphosphat; 0,15 M NaCl; 0,02 % Thimerosal; 0,1 % BSA; pH 7,3)
Schritt 4: 100 µl dieses methanolischen Puffers in einen Reaktionsnapf der Mikrotiterplatte pipettieren (beschichtet mit 150 µl Antiserum 1 : 4.000 gegen Androstenon-3-CMO-BSA)
Schritt 5: Zugabe von Androstenon-3-CMO-HRP (12 ng/25 µl Puffer)
Schritt 6: Inkubation: 30 min bei 45°C
Schritt 7: Abkippen, waschen der Platte mit Detergens (6x)
Schritt 8: Addition von 150 µl Substratpuffer
Schritt 9: Enzymreaktion: 15 min bei 15 - 20°C
Schritt 10: Abstoppen der Reaktion mit H₂SO₄, messen der Extinktion bei 450 nm
**Zu Schritt 4: Herstellung des Antiserums**

### 1. Antigensynthese

5α-Androstenon ist als Steroid zu klein, um die Bildung von Antikörpern im Organismus zu induzieren. Daher muß eine chemische Kopplung an ein großes Trägerprotein (Rinderserumalbumin = BSA) erfolgen, damit 5α-Androstenon als Antigen erkannt wird. Für die Antigensynthese werden 100 mg Androstenon in Ethanol gelöst und nach Zugabe von 150 mg O-Carboxymethyl-hydroxylamin bei pH 12 vier Stunden unter Rückfluß gekocht. Die Isolierung des entstandenen 3-Oximderivats (Androstenon-3-CMO) erfolgt nach dem Ansäuern auf pH 1 durch seine Präzipitation (der Überstand wird verworfen).

Für die Kopplung des Oximderivats an BSA werden 49,5 mg Androstenon-3-CMO zusammen mit 58 mg Carbodiimid in 1,5 ml Dioxan gelöst. Nach 35 min bei Raumtemperatur werden 44,2 mg Rinderserumalbumin in Puffer (0,04 M Na-Phosphat, 0,15 M NaCl, 0,02 % (w/v) Thimerosal, pH 7,2) tropfenweise dazugegeben. Nach 24 h bei Raumtemperatur wird das Produkt dialysiert, anschließend lyophilisiert (Ausbeute: ca. 79 mg Androstenon-3-CMO-BSA).

### 2. Immunisierung und Gewinnung des Antiserums

Dazu werden weibliche Kaninchen mit Androstenon-3-CMO-BSA immunisiert. Dabei werden jeweils 2 mg des Antigens in 1 ml Wasser aufgenommen und mit 1 ml komplettem Freund's Adjuvant vermischt (Emulsion), und in 0,5 ml Portionen verteilt auf vier Injektionsstellen (2 x subkutan, 2 x intramuskulär) verabreicht. Die Injektionen werden alle 3 Wochen wiederholt, bis sich eine ausreichende Menge Antikörper gebildet hat. Die Bestimmung der Antikörpermenge erfolgt unter Standardbedingungen. Dabei wird eine Verdünnungsreihe des Serums in Puffer erstellt und die Verdünnung ermittelt, die noch 50 % einer standardisierten Menge radioaktiv markiertes 5α-Androstenon bindet.

### 3. Isolierung der Immunglobulin G(IgG)-Fraktion aus dem Antiserum

Antikörper gegen 5α-Androstenon-3-CMO-BSA sind Bestandteil der IgG-Fraktion des Blutes und müssen für den beschriebenen enzymimmunologischen Test selektiv isoliert werden.

Dazu wird die IgG-Fraktion durch Zusatz von 180 mg Ammoniumsulfat zu 1 ml Antiserum präzipitiert. Nach 4 h bei Raumtemperatur wird das Präzipitat abzentrifugiert und nach dem Verwerfen des Überstandes in 5 ml Phosphatpuffer (siehe 1.) gelöst und gegen Wasser dialysiert. Mit dieser IgG-Fraktion (Volumen 10 ml) werden nach entsprechender Verdünnung die Vertiefungen der Mikrotiterplatten beschichtet.

### Zu Schritt 5: Synthese des Androstenon-Enzymkonjugates

Beim verwendeten Enzymimmunoassay wird 5α-Androstenon-CMO an Meerrettichperoxidase (HRP) gekoppelt. Vom Steroid-derivat werden 1 mg in 1 ml Dimethylformanid gelöst und nach Zugabe von 6,25 µl Methylmorpholin 4 min bei 0°C gerührt. Nach Temperaturerniedrigung auf -15°C werden 6,25 µl Isobutylchloroformat addiert, nach weiteren 3 min 20 mg HRP in 2 ml Wasser gelöst. Die Reaktion erfolgt bei pH 8,0 eine Stunde bei -15°C, anschließend nochmals 2 h bei 0°C. Nach Zugabe von 10 mg NaHCO₃ erfolgt eine Dialyse gegen Wasser (24 h), anschließend wird das Produkt lyophilisiert und über Sephadex^{®} G-25 gereinigt. Das Enzymkonjugat (MW ca. 44.000) eluiert mit dem Ausschlußvolumen der Säule (Proteine > 25.000 werden ausgeschlossen) und wird somit von niedermolekularen Substanzen abgetrennt (Puffersystem siehe 1.).

Anschließend erfolgt die Bestimmung der Konjugatkonzentration pro ml durch Proteinbestimmung mit HRP als Eichkurve. Durch Verdünnung mit Puffer erfolgt die Einstellung der Konjugatkonzentration für den Test (z.B. 12 ng/25 µl, siehe Schema 1, Schritt 5).

### Zu Schritt 7: Detergens

0,02 % (v/v) Tween 80 in Wasser (bidest.)

### Zu den Schritten 8 - 10: Nachweis des gebundenen Enzyms

Nach dem Abkippen und Waschen der Flatte mit Detergens (0,05 % (v/v) Tween® 80, in Wasser) erfolgt die Zugabe von Substratpuffer, bestehend aus 0,1 M Natriumacetat, 0,004 % (v/v) H₂O₂, 0,01 % (w/v) 3,3',5,5'-Tetramethylbenzidin, pH 5.

Nach 15minütiger Inkubation bei 15 - 20°C wird die Reaktion durch Addition von 25 µl 4 N Schwefelsäure abgestoppt (Schritt 10), woran sich die optische Messung des abhängig von der Enzymmenge gebildeten Farbstoffes anschließt.

## Patentansprüche

1. Verfahren zur Bestimmung von Androstenon-Gehalten in Fettgeweben, welches folgende Schritte umfaßt:
a) Verflüssigung einer Fettgewebeprobe durch Erwärmen auf eine vorgegebene Temperatur im Bereich von 45 bis 60°C;
b) Mischen einer definierten Menge des flüssigen Fetts mit einem wasserlöslichen Lösemittel für das Androstenon bei der Temperatur des flüssigen Fetts;
c) Abkühlen der Fett/Lösemittelmischung auf eine vorgegebene Temperatur, bei der wesentliche Anteile des im Lösemittel gelösten Fetts einerseits aus der Lösung ausgeschieden werden und andererseits jedoch der überwiegende Teil des in der Lösemittelphase gelösten Androstenon im Lösemittel gelöst verbleibt;
d) Entnahme einer definierten Menge der androstenonhaltigen Lösemittelphase und Verdünnung in einem vorgegebenen Verhältnis mit einer für das verwendete Nachweisverfahren verträglichen wäßrigen Pufferlösung;
e) Ermittlung des Androstenon-Gehalts der Lösemittel/Pufferlösungsphase mittels an sich bekannter kompetitiver immunologischer Nachweisreaktionen.

2. Verfahren nach Anspruch 1, dadurch gekennzeichnet, daß das Lösemittel neben guten Löseeigenschaften für das nachzuweisende Androstenon im Temperaturbereich von ca. 20 bis ca. 60°C einen Temperaturkoeffizienten für die Fettlöslichkeit aufweist, der größer ist als der Temperaturkoeffizient in diesem Temperaturbereich für die Androstenon-Löslichkeit.

3. Verfahren nach Anspruch 1 und/oder 2, dadurch gekennzeichnet, daß das Lösemittel allenfalls geringen Einfluß auf die gegebenenfalls in dem Nachweisreaktionssystem verwendeten Enzyme aufweist.

4. Verfahren nach einem oder mehreren der Ansprüche 1 bis 3, dadurch gekennzeichnet, daß das Lösemittel Antigen/Antikörperreaktionen des Nachweisreaktionssystems allenfalls geringfügig beeinflußt.

5. Verfahren nach Anspruch 4, dadurch gekennzeichnet, daß das Lösemittel bis zu 100 Vol.% Methanol umfaßt.

6. Verfahren nach einem oder mehreren der Ansprüche 1 bis 5, dadurch gekennzeichnet, daß das Mischungsverhälnis der flüssigen Fettprobe zum Lösemittel im Bereich von 0,1:10 bis 1:10 (V/V) gewählt wird.

7. Verfahren nach Anspruch 6, dadurch gekennzeichnet, daß das Mischungsverhältnis im Bereich von 0,2:10 bis 0,5:10 gewählt wird.

8. Verfahren nach einem oder mehreren der Ansprüche 1 bis 7, dadurch gekennzeichnet, daß die Verdünnung der Lösemittelphase mit Pufferlösung im Volumenverhältnis von 20:80 bis 5:95 vorgenommen wird.

9. Verfahren nach einem oder mehreren der Ansprüche 1 bis 8, dadurch gekennzeichnet, daß beim Abkühlungsschritt die Endtemperatur der Probe so gewählt wird, daß das gelöste Fett in einem solchen Umfang aus dem Lösemittel abgeschieden wird, daß im nachfolgenden Verdünnungsschritt d) eine im wesentlichen fettphasenfreie Lösung erhalten wird.

10. Verfahren nach einem oder mehreren der Ansprüche 1 bis 9, dadurch gekennzeichnet, daß im Schritt e)
(1) eine Aufnahmeplatte mit einem Antikörperserum beschichtet und die Antikörper an der Aufnahmeplatte immobilisiert werden, wobei die Antikörper spezifisch mit Androstenonen, insbesondere 5α-Androst-16-en-3-on, reagieren;
(2) die immobilisierten Antikörper mit einer definierten Menge eines enzymmarkierten Androstenons und mit einem vorgegebenen Volumen der in Schritt d) erhaltenen lösemittelhaltigen Pufferlösung bei einer Temperatur von ca. 42 bis 48°C, vorzugsweise bei ca. 45°C, für eine vorgegebene Zeitspanne gleichzeitig inkubiert werden;
(3) das nichtgebundene enzymmarkierte und freie Androstenon entfernt und die beschichtete Aufnahmeplatte gewaschen wird;
(4) eine ein Substrat enthaltende Pufferlösung auf die Aufnahmeplatte gegeben wird, wobei das Substrat mit dem Markerenzym eine Enzymreaktion eingeht;
(5) die Enzymreaktion für eine vorbestimmte Zeitspanne bei einer vorgegebenen Temperatur durchgeführt wird und danach mit einem geeigneten Reaktionsmittel abgestoppt wird; und
(6) der Reaktionsumsatz der enzymatischen Reaktion, vorzugsweise photospektrometrisch, bestimmt wird.

11. Verfahren nach Anspruch 10, dadurch gekennzeichnet, daß als Aufnahmeplatte eine Mikrotiterplatte eingesetzt wird.

12. Vorrichtung zur automatisierten Durchführung eines Verfahrens gemäß einem oder mehreren der voranstehenden Ansprüche
mit einer heizbaren Probeneingangsstation zur Aufnahme und Verflüssigung der Fettgewebeprobe;
mit einer temperierbaren, programmierbaren Pipettiervorrichtung mit mehreren Probenaufnahmevorrichtungen zur Durchführung der Verfahrensschritte b), c) und d) gemäß einem oder mehreren der Ansprüche 1 bis 9 sowie gegebenenfalls der zusätzlichen Schritte (2), (3), (4) und (5) gemäß Anspruch 10 und/oder Anspruch 11; und
mit einer Meßvorrichtung zur direkten oder indirekten Bestimmung einer den Androstenon-Gehalt der Fettgewebeprobe reflektierenden Meßgröße.

## Claims

1. Process for the determination of androstenone-contents in fatty tissues, comprising the following steps:
a) Liquefying a sample of adipose tissue by heating to a predetermined temperature in the range of from 45 to 60°C;
b) mixing a defined amount of the liquid fat with a water soluble solvent for the androstenone at the temperature of the liquid fat;
c) cooling the fat/solvent mixture to a predetermined temperature, at which considerable portions of the fat dissolved in the solvent are separated from the solution but the major part of the androstenone dissolved in the solvent phase remains dissolved in the solvent;
d) removing a defined amount of the androstenone containing solvent phase and diluting it in a predetermined ratio with an aqueous buffer solution compatible with the detection procedure;
e) determination of the androstenone content of the solvent/buffer solution phase with known competitive immunological detection reactions.

2. Process according to claim 1 characterized in that the solvent has good dissolving properties for the androstenone to be detected in the temperature range from about 20 to about 60°C and a temperature coefficient for the fat solubility which is larger than the temperature coefficient in this temperature range for the androstenone solubility.

3. Process according to one of the claims 1 and/or 2, characterized in that the solvent has at the most a small effect on the enzymes which are optionally used in the detection reaction system.

4. Process according to one or more of the claims 1 to 3, characterized in that the solvent has at the most a slight effect on antigen/antibody reactions of the detection reaction system.

5. Process according to claim 4, characterized in that the solvent comprises up to 100% by volume methanol.

6. Process according to one or more of the claims 1 to 5, characterized in that the mixing ratio of the liquid fat sample to the solvent is chosen in the range of from 0.1:10 to 1:10 ratio by volume.

7. Process according to claims 6, characterized in that the mixing ratio is chosen in the range from 0.2:10 to 0.5:10.

8. Process according to one or more of the claims 1 to 7 characterized in that the dilution of the solvent phase is carried out with a buffer solution in a ratio of from 20:80 to 5:95 by volume.

9. Process according to one or more of the claims 1 to 8, characterized in that in the cooling step the final temperature of the sample is chosen such that the dissolved fat is separated out of the solvent to such an extent that an essentially fat phase-free solution is obtained in the subsequent dilution step d).

10. Process according to one or more of the claims 1 to 9, characterized in that in step e)
(1) a receptacle plate is coated with an antibody serum, and the antibodies are immobilized on the receptacle plate, with the antibodies reacting specifically with androstenones, in particular 5α-androst-16-en-3-one;
(2) the immobilized antibodies are incubated simultaneously with a defined amount of an enzyme-labeled androstenone and with a predetermined volume of the solvent-containing buffer solution obtained in step d) at a temperature of about 42 to 48°C, preferably at about 45°C for a predetermined time;
(3) the unbound enzyme-labeled and free androstenone is removed and the coated receptacle plate is washed;
(4) a buffer solution containing a substrate is placed on the receptacle plate, whereupon the substrate enters into an enzyme reaction with the marker enzyme;
(5) the enzyme reaction is carried out for a predetermined time at a predetermined temperature and then stopped with a suitable reaction; and
(6) the reaction conversion of the enzymatic reaction is determined, preferably by photospectrometry.

11. Process according to claim 10, characterized in that as a receptacle plate a microtiter plate is used.

12. Device for carrying out a process according to one or more of the preceding claims automatically, having a heatable sample input station for receiving and liquefying the adipose tissue sample;
comprising a temperature-controllable, programmable pipetting device with a plurality of sample reception devices for carrying out process steps b), c) and d) according to one or more of the preceding claims
and, optionally, additional steps (2), (3), (4), (5) according to claims 10 and/or 11; and comprising a measuring device for direct or indirect determination of a measured variable reflecting the androstenone content of the adipose tissue sample.

## Revendications

1. Procédé pour déterminer la teneur en androsténone de tissus graisseux, qui englobe les étapes suivantes :
a) Liquéfaction d'un échantillon de tissu graisseux par chauffage à une température prédéfinie, comprise dans l'intervalle de 45 à 60°C ;
b) Mélange d'une quantité définie de la graisse liquide à un solvant soluble dans l'eau de l'androsténone à la température de la graisse liquide ;
c) Refroidissement du mélange de la graisse et du solvant à une température prédéfinie, à laquelle d'une part des parties importantes de la graisse dissoute dans le solvant se séparent de la solution, mais d'autre part la partie principale de l'androsténone dissoute dans la phase solvant reste dissoute dans le solvant ;
d) Prélèvement d'une quantité parfaitement définie de la phase solvant contenant l'androsténone, et dilution selon une proportion prédéfinie, avec une solution tampon aqueuse compatible avec le procédé de détection utilisé ;
e) Détermination de la teneur en androsténone de la phase solvant/solution tampon, grâce à des réactions de détection immunologiques compétitives connues.

2. Procédé selon la revendication 1, caractérisé en ce que le solvant, outre de bonnes propriétés de dissolution de l'androsténone à détecter, présente dans l'intervalle de températures d'environ 20 à environ 60°C un coefficient de températures, pour la solubilité des graisses, qui est supérieur au coefficient de température, dans cet intervalle de températures, pour la solubilité de l'androsténone.

3. Procédé selon la revendication 1 et/ou 2, caractérisé en ce que le solvant a tout au plus une petite influence sur les enzymes utilisées éventuellement dans le système réactionnel de détection.

4. Procédé selon l'une ou plusieurs des revendications 1 à 3, caractérisé en ce que le solvant influe tout au plus faiblement sur les réactions antigène/anticorps du système réactionnel de détection.

5. Procédé selon la revendication 4, caractérisé en ce que le solvant comporte jusqu'à 100 % en volume de méthanol.

6. Procédé selon l'une ou plusieurs des revendications 1 à 5, caractérisé en ce que le rapport de mélange de l'échantillon de graisse liquide au solvant est choisi dans l'intervalle de 0,1:10 à 1:10 (v/v).

7. Procédé selon la revendication 6, caractérisé en ce que le rapport de mélange est choisi dans l'intervalle de 0,2:10 à 0,5:10.

8. Procédé selon l'une ou plusieurs des revendications 1 à 7, caractérisé en ce que la dilution de la phase solvant par la solution tampon est réalisée selon un rapport en volume de 20:80 à 5:95.

9. Procédé selon l'une ou plusieurs des revendications 1 à 8, caractérisé en ce que, dans l'étape de refroidissement, la température finale de l'échantillon est choisie de façon que la graisse dissoute se sépare du solvant dans une mesure telle que l'on obtienne, dans l'étape de dilution d) suivante, une solution essentiellement exempte de phase graisseuse.

10. Procédé selon l'une ou plusieurs des revendications 1 à 9, caractérisé en ce que, dans l'étape e)
(1) on applique sur une plaque-support un sérum d'anticorps, et les anticorps sont immobilisés sur la plaque-support, les anticorps subissant une réaction spécifique avec des androsténones, notamment la 5α-androst-16-ène-3-one;
(2) les anticorps immobilisés sont incubés pendant un certain laps de temps, en même temps qu'une quantité définie d'une androsténone marquée par une enzyme et un volume prédéfini de la solution tampon, contenant un solvant, obtenu dans l'étape d), à une température d'environ 42 à 48°C et de préférence d'environ 45°C ;
(3) on isole l'androsténone libre et marquée par une enzyme, non liée, et on lave la plaque-support revêtue ;
(4) on place sur la plaque-support une solution tampon contenant un substrat, le substrat subissant une réaction enzymatique avec l'enzyme marqueuse ;
(5) la réaction enzymatique est mise en oeuvre pendant un laps de temps prédéterminé à une température prédéfinie, puis on y met fin à l'aide d'un réactif approprié ; et
(6) on détermine le taux de conversion de la réaction enzymatique, de préférence par photospectrométrie.

11. Procédé selon la revendication 10, caractérisé en ce que l'on utilise comme plaque support une plaque de microtitrage.

12. Dispositif pour la mise en oeuvre automatisée d'un procédé selon l'une ou plusieurs des revendications précédentes, avec une station d'entrée d'échantillons, pouvant être chauffée, destinée à recevoir et à liquéfier l'échantillon de tissu graisseux ; avec un dispositif de pipettage programmable et thermostaté, comportant plusieurs dispositifs de prélèvement d'échantillon, pour mettre en oeuvre les étapes b), c) et d), selon l'une ou plusieurs des revendications 1 à 9 et éventuellement les étapes supplémentaires (2), (3), (4) et (5) selon la revendication 10 et/ou la revendication 11 ; et un dispositif de mesure pour déterminer directement ou indirectement un paramètre qui traduit la teneur en androsténone de l'échantillon de tissu graisseux.
